# EUROPEAN PATENT SPECIFICATION

(11) **EP 0 769 951 B1**
(45) Date of publication and mention of the grant of the patent: **10.09.2003**
(21) Application number: 95923502.9
(22) Date of filing: 05.07.1995
(51) Int. Cl.: A61K 31/65, A61K 47/02, A61K 47/10, A61K 47/22, A61K 47/00

(54) **LONG-ACTING OXYTETRACYCLINE COMPOSITION**
OXYTETRACYCLIN ENTHALTENDES ARZNEIMITTEL MIT VERZÖGERTER WIRKSOFFABGABE
COMPOSITIONS A BASE D'OXYTRETRACYCLINE A ACTION PROLONGEE

(30) Priority: 09.07.1994 GB 9413873
(43) Date of publication of application: 02.05.1997
(73) Proprietor: NORBROOK LABORATORIES LIMITED, Newry BT35 6JP (GB)
(72) Inventor: PATTERSON, Alan, Belfast BT4 2NQ (GB); HOLMES, Drew, Newcastle Co Down BT33 0SL (GB)
(74) Representative: Ede, Eric
(86) International application number: GB9501583
(87) International publication number: WO96001634

(56) References cited:
- EP-A- 0 038 013
- EP-A- 0 096 942
- DE-A- 2 011 793
- FR-A- 2 081 434
- US-A- 4 772 460
- US-A- 5 075 295

## Description

This invention relates to injectable formulations containing tetracyclines, particularly oxytetracycline, which exhibit higher residual effect with less of the known detrimental effects such as pain at injection site, swelling, tissue irritancy or necrosis.

Preparation of pharmaceutical compositions containing tetracyclines and oxytetracycline in particular has always presented a challenge due to aqueous solubility constraints which firstly have impact upon composition stability, and secondly upon parenteral administration.

Prior art oxytetracycline compositions have exhibited relatively high viscosity at low temperatures which makes injection difficult, have shown poor stability and suffered limitations on strength of active principle. Thus considerable research has gone into determining suitable complexing agents and more favourable co-solvents to address these shortcomings. A review of the art suggests that presence of calcium, and especially magnesium in the formulation now appears mandatory as a complexing agent and whereas some improvements have been made in stability and delivery by adopting various co-solvent systems, higher concentration loadings and residual effect remain areas in which improvements are needed. This is especially of interest for veterinary purposes where the need is to deliver high effective doses with minimum effort in animal handling and detrimental effect on the animal requiring treatment.

At the current time prior art so-called "long-acting" oxytetracycline formulations typically contain 200 mg/ml oxytetracycline and are administered at 20 mg/kg body weight, having activity as determined by residual blood levels of oxytetracycline detectable for up to about four days or so.

An object of this invention is to provide a composition of substantially greater long acting effect whilst minimising to the greatest extent possible the defects observed in previously proposed formulations. In particular the invention to be particularly described hereinbelow provides for administration of an oxtetracycline formulation at dose rates of from 10 to 40 mg/kilogram bodyweight, giving at 30 mg/kg in animals an extended duration of effective plasma levels against susceptible organisms in excess of 9 days which is a surprising achievement in the light of the known prior art.

Solubility of oxytetracycline in non-aqueous solvents was considered by Eugene Gans and Takeru Higuchi, Journal of the American Pharmaceutical Association, 1957, *Vol XLVI*, pp 587-591.

The patent literature in this area is extensive and one could refer to the following patents which are illustrative of the decades of research carried out on formulation of tetracycline compositions:
GB-A-894 619, GB-A-1 131 007, GB-A-1 250 304, GB-A-1 286 351, GB-A-1 427 882, GB-A-1 494 558, GB-A-1 508 601, GB-A-1 514 838, GB-A-1 520 197, GB-A-1 538 903 GB-A-1 563 478, GB-A-1 592 053, GB-B-2 047 097; EP-B-38 103, EP-B-96 942; US-A-2 516 080, US-A-2 980 584, US-A-2 990 331, US-A-3 062 717, US-A-3 219 529, US-A-3 557 280, US-A-3 712 949, US-A-3 957 972, US-A-4 011 313, US-A-4 018 889, US-A-4 020 162, US-A-4 126 680, US-A-4 386 083, US-A-4 399 127, US-A-4 772 460, US-A-4 957 972, and US-A-5 075 295.

From these documents it is apparent that a variety of water-dispersible complex-stabilisers or water-miscible co-solvents have been proposed including 2-pyrrolidone, polyvinyl pyrrolidone, polyethylene glycols, caprolactam, 2-piperidone, and glycerol formal (a reaction product of glycerol and formaldehyde) in specific formulations. However it is by no means clear that the said co-solvents are equally interchangeable nor can the effect of such a change be entirely predictable for a given formulation.

US-A-4 386 083 proposes use of glycerol formal in conjunction with magnesium acetate and magnesium chloride, whilst US-A-4 772 460 proposes use of N-methylpyrrolidone (1-methyl-2-pyrrolidone) and a soluble magnesium compound. US-A-5 075 295 is particularly directed to a composition aiming to achieve up to 30% oxytetracycline, which contains polyethylene glycol 400 and magnesium oxide, but examples given only appear to show a capability of achieving up to 25% oxytetracycline and there is to applicant's knowledge no current commercially available product capable of achieving greater than 20%.

Accordingly this invention provides a composition containing as active principle a tetracycline compound, either as the free base or a salt thereof with a physiologically acceptable acid, complexed with a substantially equimolar amount of a magnesium compound, solubilised in a water miscible solvent system comprising
(i)
   a) glycerol formal in an amount of from about 10 to 50% v/v; with
   b) polyethylene glycol in an amount of from about 1 to 15% v/v;
   said composition optionally containing a pH modifier in an amount sufficient to maintain a physiochemically acceptable pH, the balance being made up with water q.s.

The composition optionally contains a thickener such as polyvinyl pyrrolidone in an amount of up to 10% w/v, and may contain usual formulation aids or auxiliaries typically used for such formulations. Thus the composition may contain antioxidants, e.g. sodium formaldehyde sulphoxylate and pH adjusting agents e.g. monoethanolamine, to provide a preferred pH range of from about 7.5 to 9.5, more preferably from about 8.5 to 9.0.

Preferably the composition contains a magnesium compound such as magnesium oxide or a salt e.g magnesium chloride.

The preferred compositions contain oxytetracycline as the base or its hydrochloride in an amount of from about 15 to 35% w/v, complexed with an equimolar ratio of a magnesium compound, preferably a salt, solubilised in a solvent system comprising polyethylene glycol in an amount of from about 1 to 15% v/v and glycerol formal in an amount of from about 10 to 50% v/v. In particular the most preferred composition contains about 30% w/v oxytetracycline, about 40% glycerol formal, about 10% v/v polyethylene glycol with a magnesium-containing complexing agent or stabiliser, antioxidant and water making up the balance. In that composition magnesium oxide is suitably present in an amount of about 2.7% w/v and, as antioxidant, sodium formaldehyde sulphoxylate in an amount of about 0.4% w/v may be used. Thus according to the present invention there is provided a formulation capable of providing from about 10 to 40 mg/kg bodyweight consisting of:

| | |
|---|---|
| Oxytetracycline | 300 mg |
| Magnesium oxide | 27 mg |
| Sodium formaldehyde sulphoxylate | 4 mg |
| Glycerol formal | 0.4 ml |
| Polyethylene glycol | 0.1 ml |
| Monoethanolamine | q.s. pH 8.6 to 8.8 |
| Water for injections | to 1 ml |

The invention will now be further described by way of example for the purposes of practical illustration only.

An oxytetracycline formulation was prepared according to the procedure indicated below using the following components:

| Active Ingredient - | |
|---|---|
| Oxytetracycline | 30% w/v |

| Excipients - | |
|---|---|
| Magnesium oxide | 2.7% w/v |
| Sodium formaldehyde sulphoxylate | 0.4% w/v |
| Glycerol formal | 40% w/v |
| Polyethylene glycol | 10% w/v |
| Monoethanolamine | q.s. pH 8.6 to 8.8 |
| Water for injections | to 100% w/v |

A controlled environment having an inert atmosphere was provided within which suitable mixing and temperature controllable heating apparatus was assembled. A nitrogen blanket is considered suitable for this purpose. The above components of the proposed composition were mixed by initially mixing a proportion of the total water with the selected solvents. The sodium formaldehyde sulphoxylate, magnesium oxide and oxytetra-cycline were added sequentially whilst mixing continuously and maintaining a temperature of approximately 65°C until all the constituents have dissolved. Thereafter, the composition is cooled to below 30°C and the pH is adjusted to lie within the range of 8.0 to 9.0, in this case by adding a sufficient amount of monoethanolamine. Finally the volume is made up with water, the pH checked and adjusted if necessary, and the composition is filtered through a 0.2 µm filter and filled into appropriate containers.

In alternative embodiments, where use of a thickener such as polyvinyl pyrrolidone is called for then it should preferably be added after the sodium formaldehyde sulphoxylate.

The following Tables provide details of Examples 1 to 14 each of which provided compositions showing excellent stability and which achieved the desired dosage levels and long acting effect.

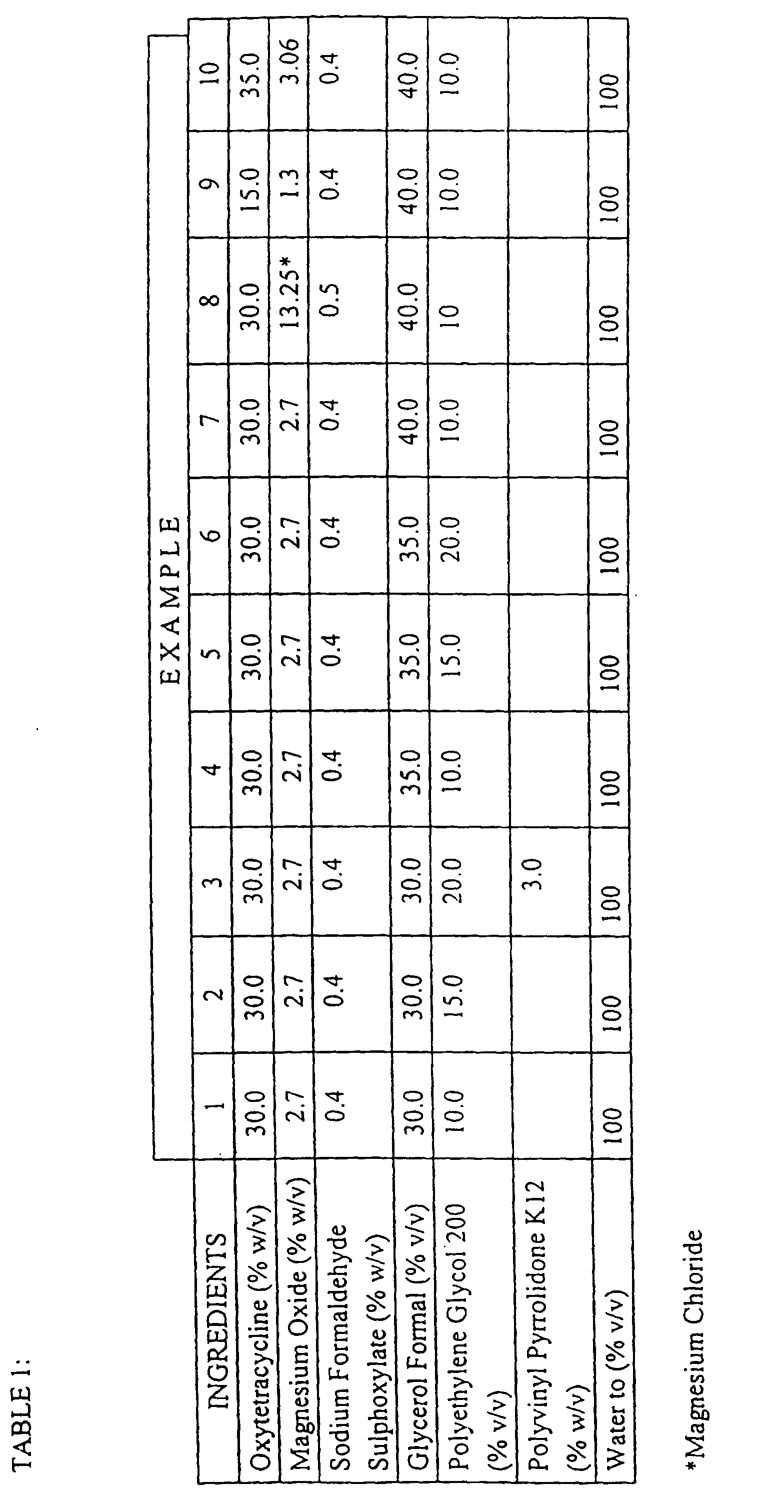

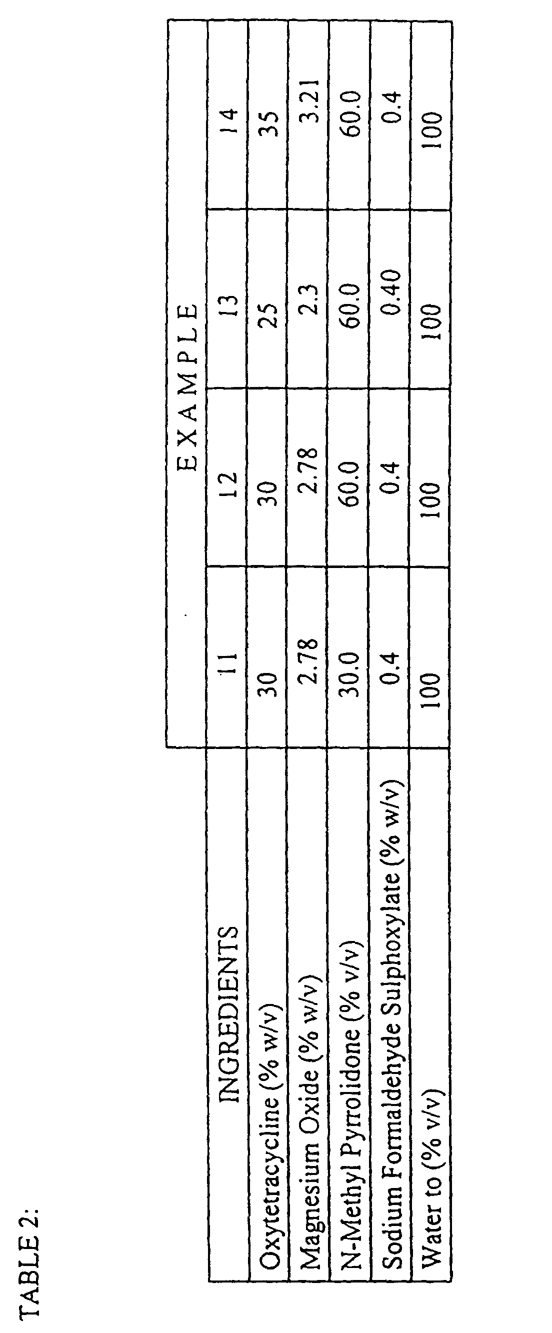

## Claims

1. A composition containing as active principle a tetracycline compound, either as the free base or a salt thereof with a physiologically acceptable acid, complexed with a substantially equimolar amount of a magnesium compound, solubilised in a water miscible solvent system comprising,
a) glycerol formal in an amount of from about 10 to 50% v/v; with
b) polyethylene glycol in an amount of from about 1 to 15% v/v;
said composition optionally containing a pH modifier in an amount sufficient to maintain a physiochemically acceptable pH, the balance being made up with water q.s.

2. A composition according to claim 1 comprising as a thickener polyvinyl pyrrolidone in an amount of up to about 10% w/v.

3. A composition according to claim 1 or claim 2 wherein the magnesium compound is magnesium oxide.

4. A composition according to claim 1 or claim 2 wherein the magnesium compound is a magnesium salt.

5. A composition according to claim 4 wherein the magnesium salt is magnesium chloride.

6. A composition according to claim 1 wherein the tetracycline compound is oxytetracycline base or its hydrochloride in an amount of from about 15 to 35% w/v.

7. A composition according to claim 1 wherein the composition contains about 30% w/v oxytetracycline, about 40% v/v glycerol formal, about 10% v/v poly-ethylene glycol with a magnesium-containing complexing agent or stabiliser, antioxidant and water making up the balance.

8. A composition according to claim 7 wherein magnesium oxide is present in an amount of about 2.7% w/v and, as antioxidant, sodium formaldehyde sulphoxylate in an amount of about 0.4% w/v may be used.

9. An injectable composition for treatment of animals which consists of:
| | |
|---|---|
| Oxytetracycline | 300 mg |
| Magnesium oxide | 27 mg |
| Sodium formaldehyde sulphoxylate | 4 mg |
| Glycerol formal | 0.4 ml |
| Polyethylene glycol | 0.1 ml |
| Monoethanolamine | q.s. pH 8.6 to 8.8 |
| Water for injections | to 1 ml, |
the said composition providing for administration of from 10 to 40 mg of oxytetracycline per kilogram of bodyweight.

10. An injectable composition for treatment of animals which comprises,
| | |
|---|---|
| Oxytetracycline (% w/v) | 30 |
| Magnesium oxide (% w/v) | 2.7 |
| Sodium formaldehyde sulphoxylate (% w/v) | 0.4 |
| Glycerol formal (% v/v) | 30 |
| Polyethylene glycol 200 (% v/v) | 10 |
| and water for injections to (% v/v) | 100 |

11. An injectable composition for treatment of animals which comprises,
| | |
|---|---|
| Oxytetracycline (% w/v) | 30 |
| Magnesium oxide (% w/v) | 2.7 |
| Sodium formaldehyde sulphoxylate (% w/v) | 0.4 |
| Glycerol formal (% v/v) | 30 |
| Polyethylene glycol 200 (% v/v) | 15 |
| and water for injections to (% v/v) | 100 |

12. An injectable composition for treatment of animals which comprises,
| | |
|---|---|
| Oxytetracycline (% w/v) | 30 |
| Magnesium oxide (% w/v) | 2.7 |
| Sodium formaldehyde sulphoxylate (% w/v) | 0.4 |
| Glycerol formal (% v/v) | 30 |
| Polyethylene glycol 200 (% v/v) | 20 |
| Polyvinyl Pyrrolidone K12 (%w/v) | 3 |
| and water for injections to (% v/v) | 100 |

13. An injectable composition for treatment of animals which comprises,
| | |
|---|---|
| Oxytetracycline (% w/v) | 30 |
| Magnesium oxide (% w/v) | 2.7 |
| Sodium formaldehyde sulphoxylate (% w/v) | 0.4 |
| Glycerol formal (% v/v) | 35 |
| Polyethylene glycol 200 (% v/v) | 10 |
| and water for injections to (% v/v) | 100 |

14. An injectable composition for treatment of animals which comprises,
| | |
|---|---|
| Oxytetracycline (% w/v) | 30 |
| Magnesium oxide (% w/v) | 2.7 |
| Sodium formaldehyde sulphoxylate (% w/v) | 0.4 |
| Glycerol formal (% v/v) | 35 |
| Polyethylene glycol 200 (% v/v) | 15 |
| and water for injections to (% v/v) | 100 |

15. An injectable composition for treatment of animals which comprises,
| | |
|---|---|
| Oxytetracycline (% w/v) | 30 |
| Magnesium oxide (% w/v) | 2.7 |
| Sodium formaldehyde sulphoxylate (% w/v) | 0.4 |
| Glycerol formal (% v/v) | 35 |
| Polyethylene glycol 200 (% v/v) | 20 |
| and water for injections to (% v/v) | 100 |

16. An injectable composition for treatment of animals which comprises,
| | |
|---|---|
| Oxytetracycline (% w/v) | 30 |
| Magnesium oxide (% w/v) | 2.7 |
| Sodium formaldehyde sulphoxylate (% w/v) | 0.4 |
| Glycerol formal (% v/v) | 40 |
| Polyethylene glycol 200 (% v/v) | 10 |
| and water for injections to (% v/v) | 100 |

17. An injectable composition for treatment of animals which comprises,
| | |
|---|---|
| Oxytetracycline (% w/v) | 30 |
| Magnesium chloride (% w/v) | 13.25 |
| Sodium formaldehyde sulphoxylate (% w/v) | 0.5 |
| Glycerol formal (% v/v) | 40 |
| Polyethylene glycol 200 (% v/v) | 10 |
| and water for injections to (% v/v) | 100 |

18. An injectable composition for treatment of animals which comprises,
| | |
|---|---|
| Oxytetracycline (% w/v) | 15 |
| Magnesium oxide (% w/v) | 1.3 |
| Sodium formaldehyde sulphoxylate (% w/v) | 0.4 |
| Glycerol formal (% v/v) | 40 |
| Polyethylene glycol 200 (% v/v) | 10 |
| and water for injections to (% v/v) | 100 |

19. An injectable composition for treatment of animals which comprises,
| | |
|---|---|
| Oxytetracycline (% w/v) | 35 |
| Magnesium oxide (% w/v) | 3.06 |
| Sodium formaldehyde sulphoxylate (% w/v) | 0.4 |
| Glycerol formal (% v/v) | 40 |
| Polyethylene glycol 200 (% v/v) | 10 |
| and water for injections to (% v/v) | 100 |

## Patentansprüche

1. Zusammensetzung mit einer Tetracyclin-Verbindung als Wirkstoff. entweder in Form der freien Base oder eines Salzes davon mit einer physiologisch unbedenklichen Säure, die mit einer im Wesentlichen äquimolaren Menge einer Magnesiumverbindung komplexiert und in einem mit Wasser mischbaren Lösungssystem aufgeschlossen ist, welches umfasst
a) Glycerolformal in einer Menge von etwa 10 bis 50 Vol.-% mit
b) Polyethylenglycol in einer Menge von etwa 1 bis 15 Vol.-%.
wobei die Zusammensetzung wahlweise einen pH-Puffer in einer Menge enthält, die zur Erhaltung eines physiologisch-chemisch unbedenklichen pHs ausreichend ist, und der Rest mit Wasser q.s. aufgefüllt wird.

2. Zusammensetzung nach Anspruch 1, mit Polyvinylpyrrolidon in einer Menge von bis zu 10 Gew./Vol.-% als Verdickungsmittel.

3. Zusammensetzung nach Anspruch 1 oder Anspruch 2, wobei die Magnesiumverbindung Magnesiumoxid ist.

4. Zusammensetzung nach Anspruch 1 oder Anspruch 2, wobei die Magnesiumverbindung ein Magnesiumsalz ist.

5. Zusammensetzung nach Anspruch 4, wobei das Magnesiumsalz Magnesiumchlorid ist.

6. Zusammensetzung nach Anspruch 1, wobei die Tetracyclin-Verbindung eine Oxytetracyclinbase oder dessen Hydrochlorid in einer Menge von etwa 15 bis 35 Gew./Vol.-% ist.

7. Zusammensetzung nach Anspruch 1, wobei die Verbindung etwa 30 Gew./Vol.-% Oxytetracyclin, etwa 40 Vol.-% Glycerolformal, etwa 10 Vol.-% Polyethylenglycol mit einem magnesiumhaltigen Komplexbilder oder einem Stabilisator, Antioxidans und als Rest Wasser enthält.

8. Zusammensetzung nach Anspruch 7, wobei Magnesiumoxid in einer Menge von etwa 2,7 Gew./Vol.-% vorhanden ist und als Antioxidans Natriumformaldehydsulfoxylat in einer Menge von etwa 0,4 Gew./Vol.-% verwendet werden kann.

9. Injizierbare Zusammensetzung zur Behandlung von Tieren. bestehend aus:
| | |
|---|---|
| Oxytetracyclin | 300 mg |
| Magnesiumoxid | 27 mg |
| Natriumformaldehydsulfoxylat | 4 mg |
| Glycerolformal | 0,4 ml |
| Polyethylenglycol | 0,1 ml |
| Monoethanolamin q.s. | pH 8.6 bis 8.8 |
| Wasser zur Injektion | bis 1 ml. |
wobei die Zusammensetzung die Gabe von 10 bis 40 mg Oxytetracyclin pro Kilogramm Körpergewicht ermöglicht.

10. Injizierbare Zusammensetzung zur Behandlung von Tieren, bestehend aus:
| | |
|---|---|
| Oxytetracyclin (Gew./Vol.-%) | 30 |
| Magnesiumoxid (Gew./Vol.-%) | 2,7 |
| Natriumformaldehydsulfoxylat (Gew./Vol.-%) | 0.4 |
| Glycerolformal (Vol.-%) | 30 |
| Polyethylenglycol 200 (Vol.-%) | 10 |
| und Wasser zur Injektion bis (Vol.-%) | 100 |

11. Injizierbare Zusammensetzung zur Behandlung von Tieren, bestehend aus:
| | |
|---|---|
| Oxytetracyclin (Gew./Vol.-%) | 30 |
| Magnesiumoxid (Gew./Vol.-%) | 2.7 |
| Natriumformaldehydsulfoxylat (Gew./Vol.-%) | 0.4 |
| Glycerolformal (Vol.-%) | 30 |
| Polyethylenglycol 200 (Vol.-%) | 15 |
| und Wasser zur Injektion bis (Vol.-%) | 100 |

12. Injizierbare Zusammensetzung zur Behandlung von Tieren, bestehend aus:
| | |
|---|---|
| Oxytetracyclin (Gew./Vol.-%) | 30 |
| Magnesiumoxid (Gew./Vol.-%) | 2.7 |
| Natriumformaldehydsulfoxylat (Gew./Vol.-%) | 0.4 |
| Glycerolformal (Vol.-%) | 30 |
| Polyethylenglycol 200 (Vol.-%) | 20 |
| Polyvinylpyrrolidon K12 (Gew./Vol.-%) | 3 |
| und Wasser zur Injektion bis (Vol.-%) | 100 |

13. Injizierbare Zusammensetzung zur Behandlung von Tieren, bestehend aus:
| | |
|---|---|
| Oxytetracyclin (Gew./Vol.-%) | 30 |
| Magnesiumoxid (Gew./Vol.-%) | 2,7 |
| Natriumformaldehydsulfoxylat (Gew./Vol.-%) | 0,4 |
| Glycerolformal (Vol.-%) | 35 |
| Polyethylenglycol 200 (Vol.-%) | 10 |
| und Wasser zur Injektion bis (Vol.-%) | 100 |

14. Injizierbare Zusammensetzung zur Behandlung von Tieren, bestehend aus:
| | |
|---|---|
| Oxytetracyclin (Gew./Vol.-%) | 30 |
| Magnesiumoxid (Gew./Vol.-%) | 2.7 |
| Natriumformaldehydsulfoxylat (Gew./Vol.-%) | 0,4 |
| Glycerolformal (Vol.-%) | 35 |
| Polyethylenglycol 200 (Vol.-%) | 15 |
| und Wasser zur Injektion bis (Vol.-%) | 100 |

15. Injizierbare Zusammensetzung zur Behandlung von Tieren, bestehend aus:
| | |
|---|---|
| Oxytetracyclin (Gew./Vol.-%) | 30 |
| Magnesiumoxid (Gew./Vol.-%) | 2,7 |
| Natriumformaldehydsulfoxylat (Gew./Vol.-%) | 0.4 |
| Glycerolformal (Vol.-%) | 35 |
| Polyethylenglycol 200 (Vol.-%) | 20 |
| und Wasser zur Injektion bis (Vol.-%) | 100 |

16. Injizierbare Zusammensetzung zur Behandlung von Tieren, bestehend aus:
| | |
|---|---|
| Oxytetracyclin (Gew./Vol.-%) | 30 |
| Magnesiumoxid (Gew./Vol.-%) | 2.7 |
| Natriumformaldehydsulfoxylat (Gew./Vol.-%) | 0.4 |
| Glycerolformal (Vol.-%) | 40 |
| Polyethylenglycol 200 (Vol.-%) | 10 |
| und Wasser zur Injektion bis (Vol.-%) | 100 |

17. Injizierbare Zusammensetzung zur Behandlung von Tieren, bestehend aus:
| | |
|---|---|
| Oxytetracyclin (Gew./Vol.-%) | 30 |
| Magnesiumchlorid (Gew./Vol.-%) | 13,25 |
| Natriumformaldehydsulfoxylat (Gew./Vot.-%) | 0.5 |
| Glycerolformal (Vol.-%) | 40 |
| Polyethylenglycol 200 (Vol.-%) | 10 |
| und Wasser zur Injektion bis (Vol.-%) | 100 |

18. Injizierbare Zusammensetzung zur Behandlung von Tieren, bestehend aus:
| | |
|---|---|
| Oxytetracyclin (Gew./Vol.-%) | 15 |
| Magnesiumoxid (Gew./Vol.-%) | 1.3 |
| Natriumformaldehydsulfoxylat (Gew./Vol.-%) | 0.4 |
| Glycerolformal (Vol.-%) | 40 |
| Polyethylenglycol 200 (Vol.-%) | 10 |
| und Wasser zur Injektion bis (Vol.-%) | 100 |

19. Injizierbare Zusammensetzung zur Behandlung von Tieren, bestehend aus:
| | |
|---|---|
| Oxytetracyclin (Gew./Vol.-%) | 35 |
| Magnesiumoxid (Gew./Vol.-%) | 3.06 |
| Natriumformaldehydsulfoxylat (Gew./Vol.-%) | 0,4 |
| Glycerolformal (Vol.-%) | 40 |
| Polyethylenglycol 200 (Vol.-%) | 10 |
| und Wasser zur Injektion bis (Vol.-%) | 100 |

## Revendications

1. Composition contenant comme principe actif un composé de tétracycline sous sa forme de base libre ou de sel de celle-ci avec un acide physiologiquement acceptable, complexé avec une quantité sensiblement équimolaire d'un composé de magnésium, solubilisé dans un système solvant miscible à l'eau constitué de,
a) glycérol formal en une quantité allant de 10 à 50 % v/v environ ; et de
b) polyéthylène glycol en une quantité allant de 10 à 15 % v/v environ ;
ladite composition contenant optionnellement un modificateur de pH dans une quantité suffisante pour maintenir un pH physiochimiquement acceptable, la différence étant constituée d'eau en quantit suffisante.

2. Composition selon la revendication 1 comprenant comme agent épaississant de la polyvinylpyrrolidone en une quantité allant jusqu'à 10 % m/v.

3. Composition selon la revendication 1 ou 2 dans laquelle le composé de magnésium est de l'oxyde de magnésium.

4. Composition selon la revendication 1 ou 2 dans laquelle le composé de magnésium est un sel de magnésium.

5. Composition selon la revendication 4 dans laquelle le sel de magnésium est du chlorure de magnésium.

6. Composition selon la revendication 1 dans laquelle le composé de tétracycline est de l'oxytétracycline base ou son chlorhydrate en une quantité allant de 15 à 35 % m/v environ.

7. Composition selon la revendication 1 dans laquelle la composition contient 30 % m/v environ d'oxytétracycline, 40 % v/v environ de glycérol formal, 10 % v/v environ de polyéthylèneglycol avec un agent de complexation ou stabilisant contenant du magnésium, un antioxydant et de l'eau complétant la différence.

8. Composition selon la revendication 7 dans laquelle l'oxyde de magnésium est présent en une quantité de 2,7 % m/v environ et comme antioxydant, on peut utiliser du formaldéhyde sulfoxylate de soude en une quantité de 0,4 % m/v environ.

9. Composition injectable pour le traitement des animaux consistant en :
| | |
|---|---|
| Oxytétracycline | 300 mg |
| Oxyde de magnésium | 27 mg |
| Formaldéhyde sulfoxylate de soude | 4 mg |
| Glycérol formal | 0,4 ml |
| Potyéthylène glycol | 0,1 ml |
| Monoéthanolamine | q.s. pH 8,6 à 8,8 |
| Eau pour préparation injectable | jusqu'à 1 ml, |
ladite composition permettant l'administration de 10 à 40 mg d'oxytétracycline par kilogramme de masse corporelle.

10. Composition injectable pour le traitement des animaux constituée de,
| | |
|---|---|
| Oxytétracycline (% m/v) | 30 |
| Oxyde de magnésium (% m/v) | 2,7 |
| Formaldéhyde sulfoxylate de soude (% m/v) | 0,4 |
| Glycérol formal (% v/v) | 30 |
| Polyéthylène glycol 200 (% v/v) | 10 |
| et eau pour préparation injectable (% v/v) | 100 |

11. Composition injectable pour le traitement des animaux constituée de,
| | |
|---|---|
| Oxytétracycline (% m/v) | 30 |
| Oxyde de magnésium (% m/v) | 2,7 |
| Formaldéhyde sulfoxylate de soude (% m/v) | 0,4 |
| Glycérol formal (% v/v) | 30 |
| Polyéthylène glycol 200 (% v/v) | 15 |
| et eau pour préparation injectable (% v/v) | 100 |

12. Composition injectable pour le traitement des animaux constituée de,
| | |
|---|---|
| Oxytétracycline (% m/v) | 30 |
| Oxyde de magnésium (% m/v) | 2,7 |
| Formaldéhyde sulfoxylate de soude (% m/v) | 0,4 |
| Glycérol formai (% v/v) | 30 |
| Polyéthylène glycol 200 (% v/v) | 20 |
| Polyvinylpyrrolidone K12 (% m/v) | 3 |
| et eau pour préparation injectable (% v/v) | 100 |

13. Composition injectable pour le traitement des animaux constituée de,
| | |
|---|---|
| Oxytétracycline (% m/v) | 30 |
| Oxyde de magnésium (% m/v) | 2,7 |
| Formaldéhyde sulfoxylate de soude (% m/v) | 0,4 |
| Glycérol formal (% v/v) | 35 |
| Polyéthylène glycol 200 (% v/v) | 10 |
| et eau pour préparation injectable (% v/v) | 100 |

14. Composition injectable pour le traitement des animaux constituée de,
| | |
|---|---|
| Oxytétracycline (% m/v) | 30 |
| Oxyde de magnésium (% m/v) | 2,7 |
| Formaldéhyde sulfoxylate de soude (% m/v) | 0,4 |
| Glycérol formai (% v/v) | 35 |
| Polyéthylène glycol 200 (% v/v) | 15 |
| et eau pour préparation injectable (% v/v) | 100 |

15. Composition injectable pour le traitement des animaux constituée de,
| | |
|---|---|
| Oxytétracycline (% m/v) | 30 |
| Oxyde de magnésium (% m/v) | 2,7 |
| Formaldéhyde sulfoxylate de soude (% m/v) | 0,4 |
| Glycérol formal (% v/v) | 35 |
| Polyéthylène glycol 200 (% v/v) | 20 |
| et eau pour préparation injectable (% v/v) | 100 |

16. Composition injectable pour le traitement des animaux constituée de,
| | |
|---|---|
| Oxytétracycline (% m/v) | 30 |
| Oxyde de magnésium (% m/v) | 2,7 |
| Formaldéhyde sulfoxylate de soude (% m/v) | 0,4 |
| Glycérol formal (% v/v) | 40 |
| Polyéthylène glycol 200 (% v/v) | 10 |
| et eau pour préparation injectable (% v/v) | 100 |

17. Composition injectable pour le traitement des animaux constituée de,
| | |
|---|---|
| Oxytétracycline (% m/v) | 30 |
| Chlorure de magnésium (% m/v) | 13,25 |
| Formaldéhyde sulfoxylate de soude (% m/v) | 0,5 |
| Glycérol formal (% v/v) | 40 |
| Polyéthylène glycol 200 (% v/v) | 10 |
| et eau pour préparation injectable (% v/v) | 100 |

18. Composition injectable pour le traitement des animaux constituée de,
| | |
|---|---|
| Oxytétracycline (% m/v) | 15 |
| Oxyde de magnésium (% m/v) | 1,3 |
| Formaldéhyde sulfoxylate de soude (% m/v) | 0,4 |
| Glycérol formai (% v/v) | 40 |
| Polyéthylène glycol 200 (% v/v) | 10 |
| et eau pour préparation injectable (% v/v) | 100 |

19. Composition injectable pour le traitement des animaux constituée de,
| | |
|---|---|
| Oxytétracycline (% m/v) | 35 |
| Oxyde de magnésium (% m/v) | 3,06 |
| Formaldéhyde sulfoxylate de soude (% m/v) | 0,4 |
| Glycérol formai (% v/v) | 40 |
| Polyéthylène glycol 200 (% v/v) | 10 |
| et eau pour préparation injectable (% v/v) | 100 |
